# EUROPEAN PATENT APPLICATION

(11) **EP 4 375 675 A1**
(43) Date of publication of application: **29.05.2024**
(21) Application number: 22846272.7
(22) Date of filing: 21.07.2022
(51) Int. Cl.: G01N 35/00, G01N 35/10, C12Q 1/686

(54) **ASSEMBLED ANALYSIS SYSTEM, METHOD, AND COMPUTER READABLE RECORDING MEDIUM**

(30) Priority: 21.07.2021 KR 20210095932; 27.08.2021 KR 20210113717
(71) Applicant: Seegene, Inc., Seoul 05548 (KR)
(72) Inventor: YOON, Su Mi, Seongnam-si Gyeonggi-do 13313 (KR); KIM, Jae Young, Seoul 07703 (KR); PARK, Sang Jong, Seoul 05059 (KR)
(74) Representative: Viering, Jentschura & Partner mbB Patent- und Rechtsanwälte
(86) International application number: PCT/KR2022/010724
(87) International publication number: WO 2023/003402

(57) **Abstract**

An assembled analysis system according to an embodiment comprises: a preparation device for preparing an analysis sample in a reaction vessel; a transfer module for transferring the reaction vessel in which the analysis sample is prepared; and a system control module for storing a file in which driving software for the preparation device is installed and state information of the transfer module is recorded. Here, the driving software is provided with an API for reading files, the driving software reads the files stored in the system control module using the API for reading files, and the state information is identified from the read file.

## Description

### [TECHNICAL FIELD]

The present disclosure relates to an assembled analysis system, method, and computer-readable recording medium.

### [BACKGROUND ART]

Nucleic acid amplification is an essential process for a method having a wider coverage in molecular biology. There are various methods for amplifying nucleic acids. For example, Miller, H. I., et al. (WO 89/06700) presents a nucleic acid sequence amplification method of hybridizing a promoter/primer sequence to a target single-stranded DNA ("ssDNA") and then transcribing many RNA copies of the sequence. Other known nucleic acid amplification methods include transcription-based amplification system (Kwoh, D. et al., Proc. Natl. Acad. Sci. U.S.A., 86:1173 (1989); and Gingeras T.R. et al., WO 88 /10315).

The most widely used method of nucleic acid amplification, known as polymerase chain reaction (hereinafter referred to as "PCR"), includes a repeated cycle process of denaturation of double-stranded DNA, annealing of oligonucleotide primers to a DNA template, and primer extension by DNA polymerase (Mullis et al., US Pat. Nos. 4,683,195, 4,683,202 and 4,800,159; Saiki et al., (1985) Science 230, 1350-1354).

PCR-based technologies have been widely used for amplification of target DNA sequences as well as scientific applications or methods in biological and medical research. These PCR-based techniques include, for example, reverse transcriptase PCR (RT-PCR), differential display PCR (DD-PCR), cloning of known or unknown genes by PCR, rapid amplification of cDNA ends (RACE), arbitrarily priming PCR (AP-PCR), multiplex PCR, SNP genome typing, and PCR-based genome analysis (McPherson and Moller, 2000) PCR. BIOS Scientific Publishers, Springer-Verlag New York Berlin Heidelberg, NY).

The PCR-based technologies described above determine the presence or absence of a target nucleic acid in a sample by amplifying and detecting a target nucleic acid of interest. In particular, when a target nucleic acid is associated with a specific disease (for example, when a target nucleic acid is derived from a specific pathogen), the presence of the target nucleic acid may help diagnose a disease risk in the subject.

In order to confirm the presence of a target nucleic acid in a sample by amplifying and detecting the target nucleic acid, a thermal cycling mechanism for amplifying the nucleic acid and an optical mechanism for detecting the amplified nucleic acid are required. In the related art, the thermal cycling mechanism was performed in a thermocycler, and the optical mechanism was performed in an electrophoresis device. Since then, with the development of RT-PCR devices capable of performing real-time polymerase chain reaction (real-time PCR), amplification and detection of nucleic acids have been performed in a single device.

In addition, in order to amplify and detect the target nucleic acid of interest, PCR-based technologies require the use of a reagent for detecting target nucleic acid including oligonucleotides (e.g., primers and/or probes), labels, DNA polymerase, dNTPs, Mg ions, and buffers that specifically hybridize to the target nucleic acid. A preprocessing process is required to prepare samples for nucleic acid amplification and detection using these target nucleic acid detection reagents. This preprocessing process is performed in a liquid handling device.

At this time, the user conducting the experiment moves the sample on which the aforementioned preprocessing has been performed from the liquid handling device to the RT-PCR device. Then, in the RT-PCR device, the target nucleic acid included in the sample is amplified, and analysis is performed on the amplified target nucleic acid.

### [DETAILED DESCRIPTION OF INVENTION]

### [TECHNICAL PROBLEMS]

According to an embodiment of the present disclosure, the present disclosure provides an assembled analysis system that may be completed by adding an additional module to the RT-PCR device and the liquid handling device described above and assembling them.

In addition, an embodiment of the present disclosure enables the aforementioned liquid handling device to determine a status of the aforementioned additional module using only a function already provided in the liquid handling device, that is, without adding new functions.

In addition, an embodiment of the present disclosure allows a file generated by the liquid handling device to be delivered to the RT-PCR device without adding a new function to functions originally supported by the aforementioned liquid handling device and RT-PCR device.

However, the problems to be solved according to the present embodiment are not limited to the above.

### [TECHNICAL SOLUTION]

An assembled analysis system according to an embodiment includes: a preparation device for preparing an analysis target sample in an analytical sample vessel; a transport module for transporting the analytical sample vessel in which the analysis target sample is prepared; and a system control module including a driving software of the preparation device and configured to store a file in which status information of the transport module is recorded.

In this case, the driving software includes a file reading application programming interface (API), the driving software reads the file stored in the system control module using the file reading API, and the status information is identified from the read file.

An assembled analysis system according to another embodiment includes: a preparation device for preparing an analysis target sample in an analytical sample vessel; an analysis device for performing an analysis operation on the prepared analysis target sample; and a system control module in which driving software of the preparation device and driving software of the analysis device are installed and a file is stored.

In this case, a file generated in the driving software of the preparation device is recorded in the system control module by a file writing API provided in the driving software of the preparation device, and the file recorded in the system control module is provided to the driving software of the analysis device by a file reading API provided in the driving software of the analysis device.

### [EFFECT OF INVENTION]

According to an embodiment, driving software of the preparation device can determine a status of the transport module that receives and transports an analytical sample vessel from the preparation device using an API included in the driving software.

In addition, according to another embodiment, a file generated by the driving software of the aforementioned preparation device can be delivered to the driving software of the analysis device that performs an analysis operation on an analysis target sample without modifying each of these driving software by using the API provided in each driving software.

### [BRIEF DESCRIPTION OF THE DRAWING]

FIG. 1 is a conceptual diagram conceptually illustrating a configuration of an assembled analysis system according to an embodiment.
FIG. 2 is a block diagram conceptually illustrating a configuration of a system control module.
FIG. 3 illustrates a direction in which an analytical sample vessel is delivered by a transport module in the assembled analysis system illustrated in FIG. 1.
FIG. 4 is a block diagram conceptually illustrating a configuration of a transport module.
FIG. 5 is a block diagram conceptually illustrating a configuration of a sealing device.
FIG. 6 is a flowchart illustrating a process in which an analytical sample vessel is delivered from a preparation device to a transport module.
FIG. 7 is a flowchart illustrating a process in which a transport command is delivered to a transport module.
FIG. 8 is a diagram illustrating a configuration of an assembled analysis system according to another embodiment.
FIG. 9 is a diagram illustrating flow of information that may be performed in an assembled analysis system according to an embodiment.

### [MODE FOR CARRYING OUT THE INVENTION]

The advantages and features of the present application, and methods of accomplishing the advantages and features will become obvious with reference to embodiments to be described below in detail along with the accompanying drawings. However, the present application is not limited to the embodiments to be disclosed below, but will be implemented in various forms different from each other. The embodiments are merely intended to make the disclosure of the present application complete and to completely notify the person with ordinary skill to which the present application pertains of the scope of the invention, and the present application is only defined by the scope of the claims.

However, in the following description, a detailed explanation of known related technologies may be omitted to avoid unnecessarily obscuring the subject matter of the present disclosure. In addition, it is understood that since terms to be described below are terms set by considering functions in the present disclosure and vary according to intention of users, such as an experimenter and a measurer or practices, the definition thereof should be based on the contents throughout this specification.

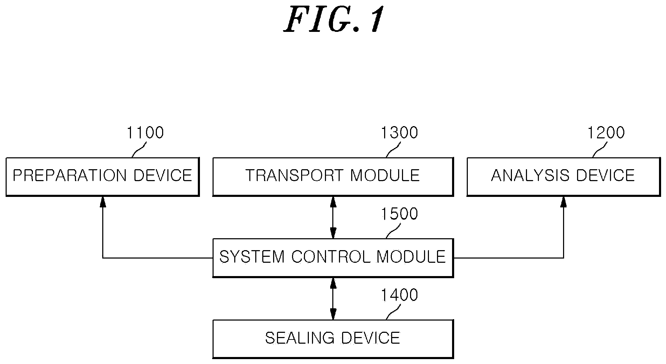

Prior to describing FIG. 1, the terms used herein will be discussed.

The term "sample" refers to a material that includes or is estimated to contain an analyte (analyte will be described later).

Such samples may include biological samples (e.g., cells, tissues, and body fluids from biological sources) or non-biological samples (e.g., food, water, and soil).

Examples of biological samples include viruses, bacteria, tissues, cells, blood (including whole blood, plasma, and serum), lymph, bone marrow fluid, sputum, swab, aspiration, bronchial lavage fluid, and bronchoalveolar lavage fluid., nasal lavage fluid, milk, urine, stool, ocular fluid, sputum, semen, brain extract, spinal fluid (SCF), j oint fluid, appendix, spleen and tonsil tissue extract, amniotic fluid or ascites. In addition, naturally occurring or synthetic nucleic acid molecules isolated from biological sources may also be included in examples of such biological samples. However, biological samples are not limited to the examples described above.

Substances used for preservation, processing, detection, etc. of the corresponding sample may be added to the aforementioned sample. For example, additional substances, such as amplification reagents, detection reagents, preservatives, water, deionized water, saline solution, pH buffer, acidic solution, and basic solution may be added to the sample. Here, the term "sample" may refer to a sample without the addition of the aforementioned additional substances as well as a sample with the addition of the aforementioned additional substances.

Meanwhile, the aforementioned sample may be referred to variously depending on the stage of molecular diagnosis. For example, the term "raw sample" may refer to a sample before being processed in a preparation device to be described below. In addition, the term "analysis target sample" may refer to intermediates including analytes prepared during the various operations of processing a sample for analysis.

Meanwhile, the aforementioned term "sample" may be used interchangeably with the term "specimen." Here, the term "specimen" itself refers to an object to be analyzed. More specifically, the term "specimen" may refer to sputum, blood, urine, stool, etc. Meanwhile, a `tested specimen' refers to a specimen classified as whether it is positive by including a specific target analyte, negative by not including a specific target analyte, or whether it is a clinical sample that should be analyzed to determine whether it includes a target analyte.

Meanwhile, analytes include antigens, antibodies, enzymes, or nucleic acids. In a specific embodiment, the analyte includes a nucleic acid. In this case, nucleic acids may be extracted through a known nucleic acid extraction process (Reference: Sambrook, J. et al., Molecular Cloning. A Laboratory Manual, 3rd ed. Cold Spring Harbor Press (2001)). The nucleic acid extraction process may vary depending on the type of sample. In addition, if the extracted nucleic acid is RNA, it may additionally undergo a reverse transcription process to synthesize cDNA (Reference: Sambrook, J. et al., Molecular Cloning. A Laboratory Manual, 3rd ed. Cold Spring Harbor Press (2001)).

The term "analytical sample vessel" refers to a sample vessel that may be accommodated in a sample holder. The analytical sample vessel accommodates a predetermined volume of the analysis target sample including a target nucleic acid or a predetermined volume of the analysis target sample not including the target nucleic acid. In addition, while the analytical sample vessel is accommodated in the sample holder, a reaction process (e.g., amplification process) or a detection process (e.g., a fluorescence signal detection process) may be performed.

The aforementioned analytical sample vessel may include a test tube, an amplification tube, a strip tube, a well plate, or a multi-well PCR plate.

Various implementation examples of the present disclosure will be described with reference to the accompanying drawings.

FIG. 1 is a block diagram conceptualizing a configuration of an assembled analysis system (or a fabricated molecular diagnostic system) 1000 according to an embodiment. Referring to FIG. 1, the assembled analysis system 1000 includes a preparation device 1100, an analysis device 1200, a transport module 1300, a sealing device 1400, and a system control module 1500. However, the block diagram illustrated in FIG. 1 is merely illustrative.

Here, the preparation device 1100 and the analysis device 1200 may be operated independently, that is, may be stand-alone devices. In addition, the respective devices 1100 and 1200 are driven by driving software. Each driving software is equipped with an application programming interface (API). This API may be used to control each of the devices 1100 and 1200 from the outside or may be used for each of the devices 1100 and 1200 to monitor an external situation using the driving software.

Here, since these devices 1100 and 1200 are medical equipment for diagnosis or examination, they may be used only after obtaining approval in certain countries. In addition, even after gaining approval, if there are changes in or features added to a main function, structure, or API of the driving software, a situation may arise where approval should be obtained again.

Meanwhile, the assembled analysis system 1000 not only includes the aforementioned stand-alone preparation device 1100 and analysis device 1200, but also includes even the transport module 1300, the sealing device 1400, and the system control module 1500 that transport or process an analytical sample vessel between the devices 1100 and 1200 or transmits and receives data therebetween. In addition, these devices 1100 and 1200 and modules 1300 to 1500 are assembled, and in that the analysis system is completed through such assembly, the analysis system according to an embodiment may be referred to as an `assemble type'.

Meanwhile, according to embodiments, the assembled analysis system 1000 may additionally include components not illustrated in FIG. 1 or may not include at least one of the components illustrated in FIG. 1. In addition, each component of the assembled analysis system 1000 may be connected differently from that illustrated in FIG. 1. Each component is described in detail hereinafter.

First, the preparation device 1100 is used in a preparation process of an analysis target sample. In this respect, the preparation device 1100 may also be referred to as a sample preparation device.

The preparation process of the analysis target sample performed by the preparation device 1100 includes, but is not limited to, a nucleic acid extraction process and a reaction mixture preparation process for nucleic acid amplification. For example, the preparation process of the analysis target sample performed by the preparation device 1100 may not include the nucleic acid extraction process according to embodiments, in which case the aforementioned nucleic acid extraction process may also be performed by other components not illustrated in FIG. 1, rather than by the preparation device 1100. However, the following description will be given on the premise that the preparation process of the analysis target sample performed by the preparation device 1100 includes the nucleic acid extraction process.

Meanwhile, since the aforementioned nucleic acid extraction process and reaction mixture preparation process for nucleic acid amplification are well-known techniques, detailed description thereof is omitted.

Referring to the preparation device 1100, the preparation device 1100 includes a pipette module and a gripper. In addition, according to embodiments, the preparation device 1100 may additionally include a dedicated module for nucleic acid extraction.

The pipette module is a module used in the aforementioned nucleic acid extraction process and reaction mixture preparation process for nucleic acid amplification. This pipette module may include a pipette tip and an arm that moves the pipette tip. The pipette module may also be referred to as a liquid handler.

The pipette module may automatically and programmatically intake and/or dispense desired quantities of reagents, analysis target samples or other liquids from designated containers for automation of chemical or biochemical laboratories.

The gripper is a module that transports the analytical sample vessel to the transport module 1300. These analytical sample vessels may contain analysis target samples. The analysis target sample accommodated in the analytical sample vessel may have undergone at least one of the aforementioned nucleic acid extraction process and the reaction mixture preparation process for nucleic acid amplification.

Meanwhile, the gripper may grip or place the analytical sample vessel, and may also move, the analytical sample vessel, while gripping the analytical sample vessel.

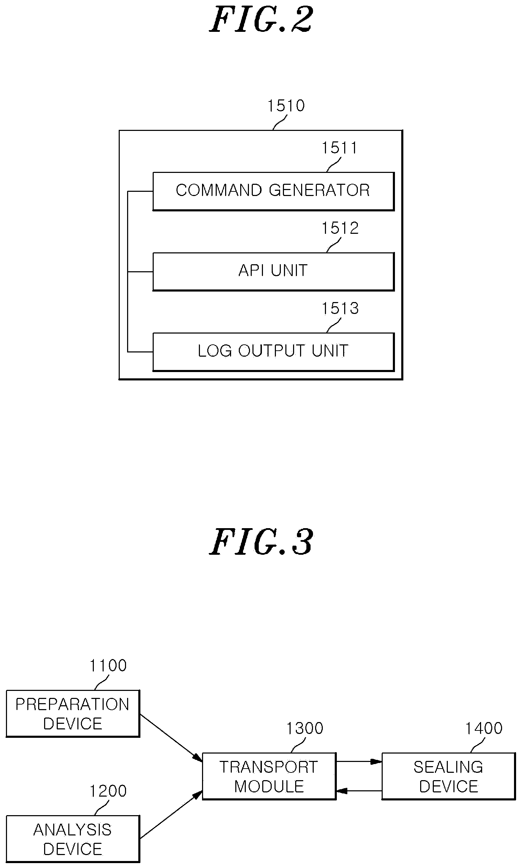

FIG. 2 is a block diagram conceptually illustrating individual modules of a driving software 1510 of the preparation device 1100. However, FIG. 3 is merely illustrative. First, this driving software 1510 is installed or stored in the system control module illustrated in FIG. 1.

Referring to FIG. 2, the driving software 1510 of the preparation device 1100 includes a command generator 1511, an API unit 1512, and a log output unit 1513. Here, each of these components is a set of programmed instructions.

In this case, the command generator 1511 is implemented to generate a control command to control hardware of the preparation device 1100.

The API unit 1512 is implemented to provide a predetermined API. In some cases, the driving software 1510 of the preparation device 1100 may monitor an external object using the API provided by the API unit 1512. Conversely, the external object may monitor or control the preparation device 1100 or the driving software 1510 of the preparation device 1100 using the API provided by the API unit 1512.

In an embodiment, the API provided by the API unit 1512 may not support a function of connecting to the transport module 1300, but support a function of recognizing a file name of a file at a predetermined position or content described in the file. In this case, 'content' includes, but is not limited to, text, images, or voices written in the file. In addition, an example of a predetermined position may include a position of a specific folder included in the system control module 1500, as is described below.

The log output unit 1513 is implemented to output a status of the preparation device 1100 and various other states as a log. Examples of output logs may include status information indicating whether the preparation device 1100 is operating normally, abnormally, or stopped, a command to call the transport module 1300, and a command to transport the analytical sample vessel to the transport module 1300, but are not limited thereto.

Referring back to FIG. 1, the analysis device 1200 is described. The analysis device 1200 is used for qualitative or quantitative analysis of an analyte. In this respect, the analysis device 1200 may be referred to as a sample analysis device.

Specifically, the analysis device 1200 performs a nucleic acid amplification process to amplify a nucleic acid having a specific nucleotide sequence and a detection process to detect the amplified nucleic acid, but is not limited thereto.

Here, the nucleic acid amplification process may be performed in various manners. For example, the nucleic acid amplification process may be performed by ligase chain reaction (LCR), see Wiedmann M, et al., "Ligase chain reaction (LCR) - overview and applications." PCR Methods and Applications 1994 Feb; 3(4): S51-64), gap filling LCR (GLCR, see WO 90/01069, European Patent No. 439182 and WO 93/00447), Qbeta replicase amplification (Qbeta, see Cahill P, et al., Clin Chem., 37(9): 1482-5 (1991), US Pat. No. 5556751), strand displacement amplification (SDA, see G T Walker et al., Nucleic Acids Res. 20(7):16911696 (1992), European Patent No. 497272), nucleic acid sequence-based amplification (NASBA, see Compton, J. Nature 350(6313):912 (1991)), transcription-mediated amplification; TMA, see Hofmann WP et al., J Clin Virol. 32(4):289-93 (2005); US Patent No. 5888779) or rolling circle amplification (RCA, see Hutchison C.A. et al., Proc. Natl Acad, Sci. USA. 102:1733217336 (2005)).

Meanwhile, since the aforementioned nucleic acid amplification process and the detection process of the amplified nucleic acid themselves are well-known techniques, a further description thereof is omitted.

The analysis device 1200 includes physically implemented equipment or structures. For example, the analysis device 1200 may include a thermocycler and an optical module. Since each of these components is known, a detailed description thereof is omitted.

Meanwhile, of course, this analysis device 1200 may also be driven or controlled by the driving software.

Referring back to FIG. 1, the transport module 1300 is described. The transport module 1300 is implemented to deliver the analytical sample vessel between the preparation device 1100, the analysis device 1200, and the sealing device 1400. FIG. 3 illustrates a direction in which the analytical sample vessel is delivered by the transport module 1300. Referring to FIG. 3, the analytical sample vessel is delivered from the preparation device 1100 to the transport module 1300, delivered from the transport module 1300 to the sealing device 1400, delivered from the sealing device 1400 to the transport module 1300, and then delivered from the transport module 1300 to the analysis device 1200. Of course, a transport path of the analytical sample vessel illustrated in FIG. 3 is merely illustrative.

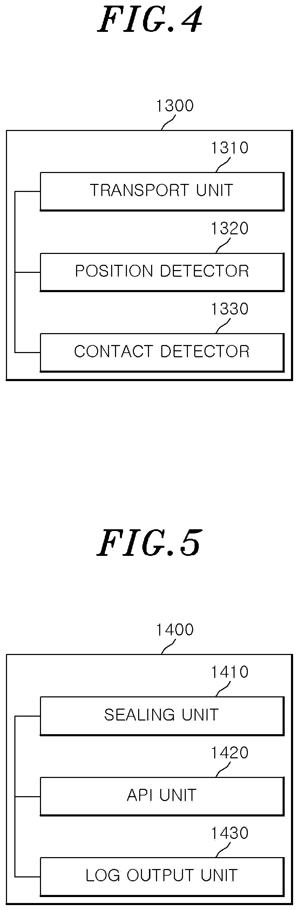

A specific configuration of this transport module 1300 is illustrated in FIG. 4. Referring to FIG. 4, the transport module 1300 includes a transport unit 1310, a position detector 1320, and a contact detector 1330, but is not limited thereto.

The transport unit 1310 is implemented to move between the preparation device 1100, the analysis device 1200, and the sealing device 1400 and deliver the analytical sample vessel therebetween. At this time, if the preparation device 1100, the analysis device 1200, and the sealing device 1400 are arranged on the same horizontal plane, the transport unit 1310 may be implemented to move in a lateral direction on the corresponding horizontal plane. In contrast, if at least two of the preparation device 1100, the analysis device 1200, and the sealing device 1400 are disposed in different positions, the transport unit 1310 may be implemented to move not only in the lateral direction but also in a longitudinal direction. In this case, since the shape of the transport unit 1310 itself is borrowed from a known one, drawings and detailed descriptions thereof are omitted.

The position detector 1320 is implemented as a position detection sensor. This position detector 1320 detects a position of the transport module 1300. As a result of the detection, the position detector 1320 may detect, for example, how far the transport module 1300 is from the preparation device 1100 or a relative position of the transport module 1300 with respect to the preparation device 1100, and provide the same.

The contact detector 1330 is implemented by a contact detection sensor. This contact detector 1330 detects whether the transport module 1300 and the analytical sample vessel are in contact with each other. As a result of the detection, the contact detector 1330 may detect and output, for example, whether the transport module 1300 and the analytical sample vessel are in contact or not.

Referring back to FIG. 1 the sealing device 1400 is described. When an analytical sample vessel including an analysis target sample is given, the sealing device 1400 is implemented to seal an inlet, for example, an upper surface of the given analytical sample vessel.

Specifically, when the sealing device 1400 receives the analytical sample vessel from the transport module 1300, the sealing device 1400 seals the upper surface thereof. In this case, a surface to be sealed may be a side surface or a lower surface, rather than the upper surface, but the description below will be given on the assumption that the surface to be sealed is the upper surface.

A specific configuration of this sealing device 1400 is illustrated in FIG. 5. Referring to FIG. 5, the sealing device 1400 includes a sealing unit 1410, an API unit 1420, and a log output unit 1430, but is not limited thereto.

As described above, the sealing unit 1410 may seal the inlet, for example, the upper surface of the analytical sample vessel including the analysis target sample. To this end, the sealing portion 1410 may adhere a transparent film to the inlet of the analytical sample vessel. At this time, for adhesion, heat or adhesive provided by the sealing unit 1410 may be used.

The API unit 1420 is implemented to provide a predetermined API. In some cases, an external object may monitor or control the status of the sealing unit 1410 using the API provided by the API unit 1420.

The log output unit 1430 is implemented to output the status of the sealing device 1400 as a log. Examples of output logs may include, but are not limited to, whether a sealing operation is currently in progress.

Referring back to FIG. 1, the system control module 1500 is described. The driving software of the aforementioned preparation device 1100 is installed in the system control module 1500. In addition, the driving software of the analysis device 1200 is installed in the system control module 1500. In addition, the system control module 1500 may store status information of the preparation device 1100 in the form of a file and also store status information of the transport module 1300 or the analysis device 1200 in the form of a file. To this end, the system control module 1500 is implemented to monitor the status of the transport module 1300.

Here, the software of each of the preparation device 1100 and the analysis device 1200 identify the status information by acquiring a file including the status information of the transport module 1300 using the API provided therein.

As described above, the API may recognize whether a file exists in a predetermined position, and if a file exists, the API may recognize a file name of the file or content described in the file. Accordingly, if the system control module 1500 reflects the monitored status of the transport module 1300 in the form of the presence or absence of a file in a predetermined position, the file name, or the content described in the file, the driving software for each of the preparation device 1100 or the analysis device 1200 may recognize the presence or absence of the file, the file name, or the content using the aforementioned API and then identify the status of the transport module 1300 therefrom.

That is, the driving software of each of the preparation device 1100 or the analysis device 1200 may identify the status of the transport module 1300 using the API recognizing the presence or absence of the file, the file name, or the content described in the file in the API provided therein.

Here, it is assumed that the software of each of the preparation device 1100 or the analysis device 1200 does not have a dedicated API for directly identifying the status of the transport module 1300. In particular, it is assumed that the preparation device 1100 and the analysis device 1200 are stand-alone devices developed to be able to operate independently, that is, having a monitoring method that may include polling and interrupt methods and delivery of the analytical sample vessel between these devices 1100 and 1200 was performed manually by people at the time when these devices 1100 and 1200 were developed and approved.

If so, there is a possibility that the transport module 1300, which will deliver the analytical sample vessel between these devices 1100 and 1200, did not exist at the time of approval or, even if it did exist, was not considered during API development. Therefore, there is a possibility that, at the time the preparation device 1100 or the analysis device 1200 was developed and approved, the API provided in the driving software of each of the devices 1100 and 1200 did not include the API for directly identifying the status of the transport module 1300.

Under this assumption, it is possible to consider adding an API for identifying the status of the transport module 1300 to the driving software of each of the preparation device 1100 or the analysis device 1200.

However, in the case of adding the API to the driving software of the preparation device 1100 or the analysis device 1200, there is the inconvenience of having to obtain approval again for the preparation device 1100 and the analysis device 1200. This is because the preparation device 1100 and the analysis device 1200 are medical or diagnostic devices used for molecular diagnosis, and when the API is added, a main function is changed in these devices 1100 and 1200 or in the driving of these devices 1100 and 1200, and therefore, approval should be obtained again.

Accordingly, in an embodiment, a technology in which the driving software of the preparation device 1100 or the analysis device 1200 identify the status of the transport module 1300 using only the existing API, without an additional API, and thus, without the need to obtain approval again. Hereinafter, the system control module 1500, which is one of the components that makes this technology available, is described.

The system control module 1500 controls the transport module 1300, the sealing device 1400, or the analysis device 1200. Specifically, the system control module 1500 may control the movement or motion of the transport module 1300 as illustrated in FIG. 3. In addition, the system control module 1500 may control the sealing device 1400 to perform a sealing operation. In addition, the system control module 1500 may control the aforementioned door of the analysis device 1200 to be opened or closed.

In addition, the system control module 1500 may identify the status of the transport module 1300. This is described more detail.

First, the system control module 1500 identify the position of the transport module 1300. To this end, the system control module 1500 obtains the position of the transport unit 1310 detected by the position detector 1320 from the position detector 1320. From the obtained position, when it is determined that the transport unit 1310 is close to the preparation device 1100 within a certain distance, the system control module 1500 identifies that 'the transport module 1300 is ready to receive the analytical sample vessel from the preparation device 1100.

In addition, the system control module 1500 identify whether the transport module 1300 has received the analytical sample vessel from the preparation device 1100. To this end, the system control module 1500 obtains information on whether the transport module 1300 and the analytical sample vessel are in contact with each other from the contact detector 1330. From the obtained information, when it is determined that the transport module 1300 and the analytical sample vessel are in contact with each other, the system control module 1500 identifies that 'the transport module 1300 has received the analytical sample vessel from the preparation device 1100'.

In addition, the system control module 1500 identifies the status of the sealing device 1400 or the analysis device 1200. To this end, the system control module 1500 may monitor a log output by the log output unit 1430 of the sealing device 1400 or a log output by the driving software of the analysis device 1200. The status or log identified in this manner is status information and may be generated in the form of a file and stored in a specific position.

In addition, the system control module 1500 identifies the status or the log of the preparation device 1100. To this end, the system control module 1500 may be connected to the driving software of the preparation device 1100 and may identify the status or log therefrom. The status or log identified in this manner may be status information and may be generated in the form of a file and stored in a specific position.

In addition, the system control module 1500 generates and stores the status of the transport module 1300 in the form of a file as status information. In addition, the system control module 1500 may generate the status or log of the sealing device 1400 or the status or log of the analysis device 1200 as status information in the form of a file and store the same in a specific position. The presence of the file, the file name of the file, or the content of the file may change depending on the status information of the transport module.

Here, the driving software 1510 of the preparation device 1100 or the driving software of the analysis device 1200 already knows the position in which the aforementioned file is stored. Accordingly, based on the information on the position, the position may be accessed to read the file. A method used in this case is a file reading API. That is, the driving software for each of the preparation device 1100 and the analysis device 1200 includes the file reading API.

Meanwhile, the driving software 1510 of the preparation device 1100 should be able to identify whether the transport module 1300 is ready to receive the analytical sample vessel from the preparation device 1100. This is because the preparation device 1100 cannot deliver the analytical sample vessel to the transport module 1300 if the transport module 1300 is not ready to receive the analytical sample vessel. Hereinafter, an operation of the preparation device 1100 to identify whether the transport module 1300 is ready to receive the analytical sample vessel is referred to as a first operation of the preparation device 1100.

In addition, even after the preparation device 1100 identifies the status of the transport module 1300, the preparation device 1100 should identify whether the transport module 1300 has received the analytical sample vessel from the preparation device 1100. This is because the operations that the preparation device 1100 may perform may, of course, vary depending on a specific operation sequence, but may include operations that may be performed only after it is determined that the transport module 1300 has received the analytical sample vessel. Hereinafter, the operation of determining whether the transport module 1300 has received the analytical sample vessel is referred to as a second operation of the preparation device 1100.

Hereinafter, an example in which the preparation device 1100 implements the aforementioned first and second operations is described in detail.

First, a method in which the preparation device 1100 performs the first situation is described.

The system control module 1500 controls the transport unit 1310 of the transport module 1300. Specifically, the transport unit 1310 approaches the preparation device 1100 by the system control module 1500.

In addition, the system control module 1500 detects the position of the approaching transport unit 1310. To this end, the system control module 1500 obtains the position of the transport unit 1310 detected by the position detector 1320 from the position detector 1320. When the transport unit 1310 comes to a predetermined position, for example, a position closest to the preparation device 1100, the system control module 1500 identifies that 'the transport module 1300 is ready to receive the analytical sample vessel from the preparation device 1100.' To this end, the system control module 1500 may determine whether the obtained position of the transport unit 1310 is the predetermined position or a position closer to the predetermined position based on the position detected by the position detector 1320, and the technology of this portion is well known, and thus, a detailed description thereof is omitted.

If it is identified that 'the transport module 1300 is ready to receive the analytical sample vessel from the preparation device 1100,' the system control module 1500 generates the status of the transport module 1300 as status information in the form of a file and stores the same in a specific position. The form of the status information may be a file name of the file or content, such as text, image, or voice written in the file.

An example of a specific form of status information is described. The system control module 1500 generates a file with the file name 'a.txt' and stores the generated file in a specific position.

Alternatively, the system control module 1500 may write text `transport unit ready' in a file called 'a.txt' and then store the file. This file name or text in the file means 'the transport module 1300 is ready to receive the analytical sample vessel from the preparation device 1100'.

The driving software 1510 of the preparation device 1100 reads the aforementioned file using the file reading API. Status information is identified from the read file. In this case, the operation using the file reading API may be performed repeatedly until a file exists in the corresponding position or until the target file name or content of the file is recognized. The presence of a file may be recognized by polling and interrupt methods.

If the recognized file name is 'a.txt' or `transport unit ready' is included in the contents described in the file, the driving software 1510 of the preparation device 1100 identifies that 'the transport module 1300 is ready to receive the analytical sample vessel from the preparation device 1100.

Then, the system control module 1500 drives the preparation device 1100. Specifically, the system control module 1500 drives the gripper in the preparation device 1100 to deliver the analytical sample vessel to the transport unit 1310 of the transport module 1300.

That is, according to an embodiment, the driving software of the preparation device 1100 may obtain the file in which the status of the transport module 1300 that receives the analytical sample vessel from the preparation device 1100 and transports the same is recorded, using the file reading API.

Next, a method in which the preparation device 1100 performs the second situation is described.

After the transport module 1112 moves toward the transport unit 1310, the driving software 1510 of the preparation device 1100 repeatedly identifies whether the file is stored in the system control module 1500 using the API provided by the API unit 1512, that is, the file reading API. Whether the transport unit 1310 of the transport module 1300 has actually received the analytical sample vessel from the preparation device 1100 is identified from the presence of the file, the file name of the file, or the content of the file.

Meanwhile, the system control module 1500 identifies whether the transport unit 1310 of the transport module 1300 and the preparation device 1100 are in contact with each other. To this end, the system control module 1500 identifies whether the transport unit 1310 and the preparation device 1100 are in contact with each other from the contact detector 1330. If contact therebetween is identified, the system control module 1500 identifies that 'the transport unit 1310 has received the analytical sample vessel from the preparation device 1100.'

Then, the system control module 1500 generates a file. In this file, the aforementioned status information is reflected in the file name or the content of the file.

Then, the driving software 1510, which has previously repeatedly identified the file, identifies that the transport unit 1310 of the transport module 1300 has received the analytical sample vessel from the preparation device 1100 from the file stored in the system control module 1500.

Then, the driving software 1510 issues a command to the transport unit 1310 of the transport module 1300 to move to the sealing device 1400, while gripping the analytical sample vessel. The command given in this manner is monitored by the system control module 1500, and a monitoring result is reflected in the form of a file in the system control module 1500, and based on this file, the transport unit 1310 of the transport module 1300 is controlled to move the analytical sample vessel to the sealing device 1400 in a gripped state.

Meanwhile, so far, the first and second operations for the preparation device 1100 to identify the status of the transport module 1300 has been described. Hereinafter, a method in which the system control module 1500 identifies the status of the preparation device 1100 is described. There are several methods, and any one of these methods may be adopted, but it is not limited thereto.

Among these, a first method is described. The driving software 1510 of the preparation device 1100 outputs the status of the preparation device 1100 in the form of a log.

The output log is reflected in the system control module 1500 in the form of a file.

The system control module 1500 may identify the status of the preparation device 1100 from the file.

Next, the second method is described. The driving software 1510 of the preparation device 1100 outputs the status of the preparation device 1100 in the form of a log.

The system control module 1500 directly obtains the output log and identifies the status of the preparation device 1100 therefrom.

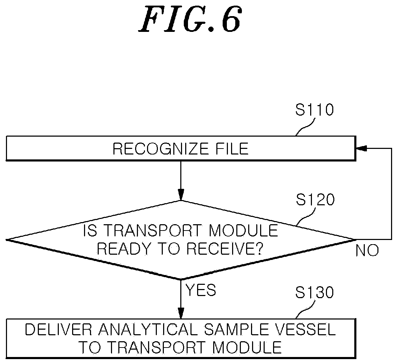

FIG. 6 is a flowchart of a process in which an analytical sample vessel is delivered from a preparation device to a transport module. However, since FIG. 6 is merely illustrative, the present disclosure is not limited to that illustrated in FIG. 6.

Referring to FIG. 6, the driving software 1510 recognizes the file stored in the system control module 1500 using the file reading API provided by the API unit 1512 (S110). At this time, according to embodiments, the driving software 1510 may recognize the presence of a file, the file name, or the content described in the file.

From the recognized file, the driving software 1510 identifies whether the transport module 1300 is ready to receive the analytical sample vessel from the preparation device 1100 (S120).

If it is identified that the transport module 1300 is not ready, S110 is performed again and the operations after S 110 illustrated in FIG. 6 are performed.

However, when it is determined that the transport module 1300 is completely ready, the driving software 1510 drives the gripper gripping the analytical sample vessel, so that the gripper moves toward the transport unit 1310 of the transport module 1300. Then, the analytical sample vessel is delivered to the transport unit 1310 of the transport module 1300 (S130).

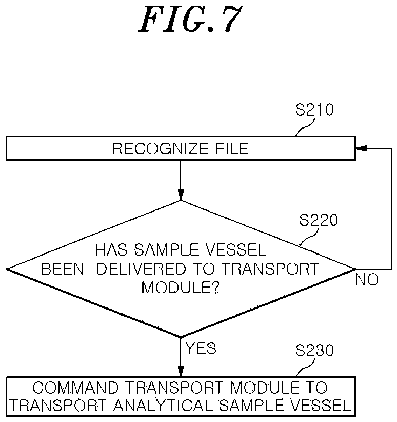

FIG. 7 is a flowchart of a process in which a transport command is delivered to a transport module. However, since FIG. 7 is merely illustrative, the present disclosure is not limited to that illustrated in FIG. 7.

Referring to FIG. 7, the driving software 1510 recognizes a file stored in the system control module 1500 using the file reading API provided by the API unit 1512 (S210). At this time, according to embodiments, the driving software 1510 may recognize the presence of a file, the file name, or the content described in the file.

From the recognized file, the driving software 1510 determines `whether the transport module 1300 has received the analytical sample vessel from the preparation device 1100' (S220).

If it is determined that transport module 1300 is not ready, S210 is performed again and the operations after S210 illustrated in FIG. 7 are performed.

However, when it is determined that transport module 1300 is completely ready, the driving software 1510 issues a command to the transport module 1300 to move to the sealing device 1400 while gripping the analytical sample vessel (S230). The command issued in this manner is monitored by the system control module 1500, a monitoring result is generated as a file, and the system control module 1500 controls the transport unit 1310 of the transport module 1300 to move to the sealing device 1400, while griping the analytical sample vessel, from the file.

Meanwhile, each method illustrated in FIGS. 6 and 7 may be implemented in a computer-readable recording medium that stores a computer program programmed to perform the operations included in each of these methods, or may be implemented in the form of a computer program programmed to perform each operation included in the method and stored in the computer-readable recording medium.

Hereinafter, other embodiments of the present disclosure are described.

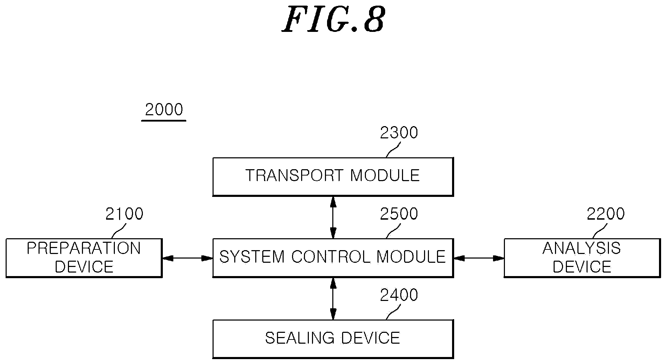

FIG. 8 is a block diagram conceptualizing a configuration of a fabricated molecular diagnostic system (or an assembled analysis system) 2000 according to another embodiment. Referring to FIG. 8, the assembled analysis system 2000 includes a preparation device 2100, an analysis device 2200, a transport module 2300, a sealing device 2400, and a system control module 2500. However, the block diagram illustrated in FIG. 8 is merely illustrative.

Here, the preparation device 2100 and the analysis device 2200 may be operated independently, that is, may be stand-alone devices. In addition, the driving software of each of the devices 2100 and 2200 includes an API. The API may be used to control or monitor each of the devices 2100 and 2200 from the outside or may be used for the driving software of each of the devices 2100 and 2200 to monitor an external situation or control or monitor an external device.

Here, since these devices 2100 and 2200 are medical equipment for diagnosis or examination, they may be used only after obtaining approval in certain countries. In addition, even after gaining approval, if there are changes in or features added to a main function, or structure, a situation may arise where approval should be obtained again. Since the API corresponds to the main function of these devices 2100 and 2200, when the API is changed or added, a situation may arise in which approval should be obtained again.

Meanwhile, the assembled analysis system 2000 not only includes the aforementioned stand-alone preparation device 2100 and analysis device 2200, but also includes even the transport module 2300, the sealing device 2400, and the system control module 2500 that deliver or process an analytical sample vessel between the devices 2100 and 2200 or transmits and receives data therebetween. In addition, these devices 2100 and 2200 and modules 2300 to 2500 may be combined with each other through assembly, and in that the analysis system is completed through such assembly, the analysis system according to an embodiment may be referred to as an `assemble type'.

Meanwhile, according to an embodiment, the assembled analysis system 2000 may additionally include components not illustrated in FIG. 8 or may not include at least one of the components illustrated in FIG. 8. In addition, each component of the assembled analysis system 2000 may be connected differently from that illustrated in FIG. 1. Each component is described in detail hereinafter.

First, the preparation device 2100 is used to prepare an analysis target sample.

The preparation operation of the analysis target sample performed by the preparation device 2100 includes, but is not limited to, a nucleic acid extraction operation and a reaction mixture preparation operation for nucleic acid amplification. For example, the preparation operation of the analysis target sample performed by the preparation device 2100 may not include the nucleic acid extraction operation according to embodiments, in which case the aforementioned nucleic acid extraction operation may also be performed by other components not illustrated in FIG. 8, rather than by the preparation device 2100. However, the following description will be given on the premise that the preparation operation of the analysis target sample performed by the preparation device 2100 includes the nucleic acid extraction operation.

Meanwhile, since the aforementioned nucleic acid extraction operation and reaction solution preparation operation for nucleic acid amplification are known techniques, a detailed description thereof is omitted.

The preparation device 2100 includes equipment or a structure that is physically implemented. In addition, a driving software 2510 of the preparation device may generate a command for driving, drive the equipment or structure based on the generated command, output a driving result and various other messages or information as a log, and provide a certain API.

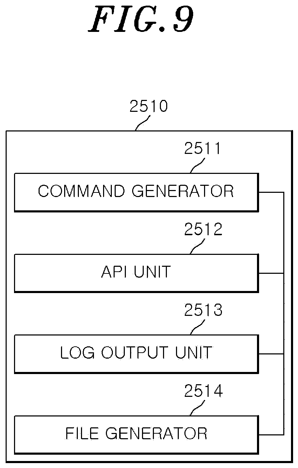

FIG. 9 is a block diagram conceptually illustrating a configuration of the driving software 2510 of the preparation device 2100. However, FIG. 3 is merely illustrative. The driving software 2510 may be installed in the system control module 2500.

Referring to FIG. 9, the driving software 2510 of the preparation device 2100 includes a command generator 2511, an API unit 2512, a log output unit 2513, and a file generator 2514.

Here, the command generator 2511 is implemented to generate a control command for controlling the preparation device 2100.

The API unit 2512 is implemented to provide a predetermined API. In some cases, the preparation device 2100 may monitor or control an external object using the API provided by the API unit 2512. Conversely, the external object may monitor or control the preparation device 2100 using the API provided by the API unit 2512.

In an embodiment, the API provided by the API unit 2512 does not support a function of accessing the analysis device 2200, which is described below. However, the corresponding API supports a function of generating a file having a specific file name and a function of writing the generated file to a predetermined position. For example, a file writing API may be included in the API supported by the API unit 2512.

In this case, the file may include certain content. Here, 'content' includes, but is not limited to, text, images, or voices written in the file. In addition, an example of a predetermined position may be a file storage constituting the system control module 2500, as is described below, and the position may be a position agreed upon in advance with the analysis device 2200.

The log output unit 2513 is implemented to output a status of the preparation device 2100 and various other states as a log. Examples of output logs may include status information indicating whether the preparation device 2100 is operating normally, abnormally, or stopped, a command to call the transport module 2300, and a command to transport the analytical sample vessel to the transport module 2300, but are not limited thereto.

The file generator 2514 is implemented to generate a file. The file includes an instruction that the analysis device 2200 refers to when performing analysis on target nucleic acid molecules.

The instruction include, for example, at least one of an analysis protocol for an analysis target sample or an amplification protocol used for amplification, information on a plate with a plurality of wells, information on a tested specimen included in each of the plurality of wells, information on a reagent included in each of the plurality of wells, information on an optical scan method performed during the analysis, and a reaction condition during the analysis, but is not limited thereto. Here, the 'reaction condition' described above may be, for example, a PCR protocol, but are not limited thereto. In addition, the tested specimen refers to a specimen for which it is known or recognized whether the specimen includes a target analyte (positive), does not contain the target analyte (negative), or is a clinical sample.

When the file generator 2514 generates the aforementioned file, the preparation operation for the target nucleic acid molecule performed by the preparation device 2100 may be the basis. In other words, the contents of the aforementioned instruction may be determined depending on whether on which plate the pipette module of the preparation device 2100 performed preparation operation, which specimens are included in each of a plurality of wells included in the corresponding plate, or which reagent was dispensed into each of a plurality of wells. In this case, the subject that specifically determines the contents of the instruction may be the file generator 2514. To this end, in the file generator 2514, a table in which contents of an instruction for a preparation operation are written may be prepared in advance. In contrast, a subject that specifically determines the contents of the instruction may be an operator of the assembled analysis system 2000 according to an embodiment example.

Meanwhile, the aforementioned file generated by the file generator 2514 may be recorded in a certain position by the function of writing a file to a predetermined position, that is, the file writing API, among the API functions provided by the aforementioned API unit 2512.

In addition, the file generator 2514 may record the file name of the aforementioned file in a predetermined position using the API function provided by the aforementioned API unit 2512. In this case, the predetermined position in which the file name is recorded may be a specific folder of the system control module 2500, as is described below.

Referring back to FIG. 8, the analysis device 2200 is described. The analysis device 2200 is used for qualitative or quantitative analysis of an analyte.

Specifically, the analysis device 2200 performs a nucleic acid amplification operation to amplify a nucleic acid having a specific nucleotide sequence and an analysis operation to detect the amplified nucleic acid, but is not limited thereto.

Here, the nucleic acid amplification operation may be performed in various manners. For example, the nucleic acid amplification process may be performed by ligase chain reaction (LCR), see Wiedmann M, et al., "Ligase chain reaction (LCR) - overview and applications." PCR Methods and Applications 1994 Feb; 3(4): S51-64), gap filling LCR (GLCR, see WO 90/01069, European Patent No. 439182 and WO 93/00447), Qbeta replicase amplification (Qbeta, see Cahill P, et al., Clin Chem., 37(9): 1482-5 (1991), US Pat. No. 5556751), strand displacement amplification (SDA, see G T Walker et al., Nucleic Acids Res. 20(7):16911696 (1992), European Patent No. 497272), nucleic acid sequence-based amplification (NASBA, see Compton, J. Nature 350(6313):912 (1991)), transcription-mediated amplification; TMA, see Hofmann WP et al., J Clin Virol. 32(4):289-93 (2005); US Patent No. 5888779) or rolling circle amplification (RCA, see Hutchison C.A. et al., Proc. Natl Acad, Sci. USA. 102:1733217336 (2005)).

Meanwhile, since the aforementioned nucleic acid amplification operation and the analysis operation of the amplified nucleic acid itself are known techniques, a further description thereof is omitted.

The analysis device 2200 includes physically implemented equipment or structure. In addition, the driving software the analysis device 2200 is installed in the system control module 2500 and controls the equipment or structure implemented in the analysis device 2200. In addition, a certain API may be provided in the driving software.

The driving software of the analysis device 2200 drives the analysis device 2200 based on the file generated by the file generator 2514. For example, a thermocycler and optical module included in the analysis device 2200 may be driven by the file.

The driving software has an API. This API may also be used to control an operation of a door (not illustrated in the drawing) provided in the analysis device 2200.

In addition, the aforementioned API may support a function of reading the file itself, the file name of the file, or the content described in the file at a predetermined position. That is, the file reading API may be included therein.

In this case, 'content' includes, but is not limited to, text, images, or voices written in the file. In an embodiment, the presence of a file stored in the system control module 2500, the file name, or the content of the file may be identified or recognized by the file reading API. An object read in this manner may include various things. For example, the object may include a file generated by the file generator 2514 of the preparation device 2100, that is, a file including an instruction that the analysis device 2200 refers to when performing an analysis operation.

Referring back to FIG. 8, the transport module 2300 is described. The transport module 2300 is implemented to deliver the analytical sample vessel between the preparation device 2100, the analysis device 2200, and the sealing device 2400. Regarding this transport module 2300, those described above with reference to FIG. 3 are referred to.

Referring back to FIG. 8, the sealing device 2400 is described. This sealing device 2400 is implemented to seal an inlet, for example, an upper surface of the analytical sample vessel accommodating the analysis target sample. Regarding the sealing device 2400, those described above with reference to FIG. 5 are referred to.

Referring back to FIG. 8, the system control module 2500 is described.

When the driving software 2510 of the preparation device 2100 generates a file, the file is recorded in the system control module 2500 by the file writing API. Then, the driving software of the analysis device 2200 reads the file recorded in the system control module 2500 from the system control module 2500 using the file reading API.

That is, the driving software of the preparation device 2100 and the driving software of the analysis device 2200 may exchange files with each other by using the file writing API that writes a file to a predetermined position or the file reading API that reads a file from a predetermined position, among APIs provided therein.

Referring to FIG. 8, the system control module 2500, like the system control module 2500 illustrated in FIG. 1, may be implemented using a processor and a memory that stores an instruction executable by the processor.

Specifically, the system control module 2500 stores a file generated by the driving software of the preparation device 2100. As described above, the stored file may include an instruction that the analysis device 2200 refers to when performing analysis on the target nucleic acid molecule. At this time, the position may be a pre-agreed position, for example, a specific folder on a computer.

Meanwhile, the system control module 2500 controls the transport module 2300, the sealing device 2400, or the analysis device 2200. Specifically, the system control module 2500 may control the transport module 2300 to approach the preparation device 2100. In addition, the system control module 2500 may control the transport module 2300, which has received the analytical sample vessel, to move toward the sealing device 2400. Alternatively, the system control module 2500 may control the aforementioned door of the analysis device 2200 to open or close.

In addition, the system control module 2500 may control movement or motion of the transport module 2300. In addition, the system control module 2500 may control the sealing device 2400 to perform a sealing operation.

In addition, the system control module 2500 identifies a status of the transport module 2300. In addition, the system control module 2500 identifies whether the transport module 2300 has received the analytical sample vessel from the preparation device 2100. In addition, the system control module 2500 identifies the status of the sealing device 2400 or the analysis device 2200.

Meanwhile, the system control module 2500 may include a storage space on the computer in which a predetermined file may be stored. The storage space has a specific address.

In this case, the system control module 2500 or the driving software of the preparation device or the analysis device already knows these addresses. Accordingly, based on the address, the system control module 2500 or each driving software may record, read, or recognize the file that each driving software generates at the corresponding position.

Hereinafter, a specific implementation example in which a file is delivered from the preparation device 2100 to the analysis device 2200 is described.

The preparation device 2100 generates a file (S10). Specifically, the file generator 2514 of the preparation device 2100 generates a file. The file includes an instruction that the analysis device 2200 refers to when performing analysis on target nucleic acid molecules. The instruction may include various information described above, and the description thereof will refer to that given above.

The file generated in S10 is recorded or stored in the system control module 2500 (S11). Among the API functions provided by the API unit 2512 of the driving software 2510 of the preparation device 2100, the function of writing a file to a predetermined position, that is, the file writing API, may be used for recording or storage. In addition, the position in which the file is recorded or stored may be a position agreed upon in advance.

Then, the driving software of the analysis device 2200 reads the corresponding file using the file reading API (S12). To this end, the driving software of the analysis device 2200 may monitor whether a new file is recorded or stored in the file storage 1521. Monitoring methods may include a polling method and an interrupt method.

Thereafter, analysis of the target nucleic acid molecule is performed by referring to the file read in S12 of the analysis device 2200 (S13).

That is, according to an embodiment, the driving software of the preparation device 2100 or the driving software of the analysis device 2200 may exchange files with each other by using the API that records a file in a predetermined position or reads a file from a predetermined position, among the APIs provided therein.

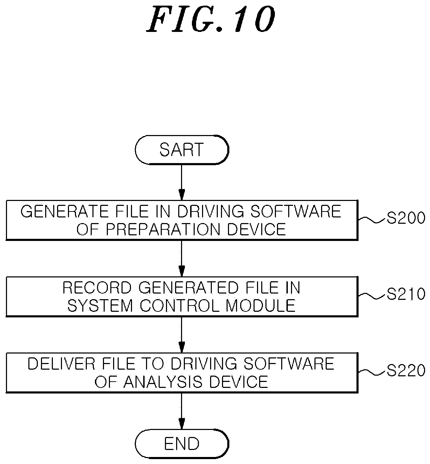

FIG. 10 is a flowchart of a file information method that may be performed in the assembled analysis system 2000 according to another embodiment. However, since FIG. 10 is merely illustrative, the scope of the present disclosure is not limited to that illustrated in FIG. 10.

Referring to FIG. 10, in the file delivery method, a file is generated in the driving software of the preparation device 2100 (S200).

Thereafter, the file generated in operation S200 is stored or recorded in the system control module 2500 (S210) and then delivered to the driving software of the analysis device 2200 (S220).

Meanwhile, the method illustrated in FIG. 10 may be implemented in a computer-readable recording medium that stores a computer program programmed to perform the operations included in each of these methods, or may be implemented in the form of a computer program programmed to perform each operation included in the method and stored in the computer-readable recording medium.

The above description is merely illustrative of the technical idea of the present invention, and various modifications, changes, and substitutions may be made by those skilled in the art without departing from the essential characteristics of the present invention. Accordingly, the exemplary embodiments disclosed in the present invention and the accompanying drawings are not intended to limit the technical idea of the present invention but to describe the present invention, and the scope of the technical idea of the present invention is not limited by the exemplary embodiments and the accompanying drawings. The protection scope of the present invention should be interpreted by the following claims, and all technical ideas within the equivalent scope should be interpreted as being included in the scope of the present invention.

### CROSS-REFERENCE TO RELATED APPLICATION

This patent application claims priority under Article 119(a) of the U.S. Patent Act (35 U.S.C §119(a)) for Korean Patent Application No. 10-2021-0095932 filed in Korea on July 21, 2021 and Korean Patent Application No. 10-2021-0113717 filed in Korea on August 27, 2021. All contents are hereby incorporated by reference into this patent application. In addition, this patent application claims priority for countries other than the United States for the same reasons as above, and the entire contents thereof are incorporated into this patent application by reference.

## Claims

1. An assembled analysis system comprising:
a preparation device for preparing an analysis target sample in an analytical sample vessel;
a transport module for transporting the analytical sample vessel in which the analysis target sample is prepared; and
a system control module including a driving software of the preparation device and configured to store a file in which status information of the transport module is recorded,
wherein the driving software includes a file reading application programming interface (API),
the driving software reads the file stored in the system control module using the file reading API, and
the status information is identified from the read file.

2. The assembled analysis system of claim 1, wherein the preparation device prepares the analysis target sample by dispensing at least one of a specimen and a reagent and accommodates the prepared analysis target sample in the analytical sample vessel.

3. The assembled analysis system of claim 1, wherein the driving software includes no API for directly identifying a status of the transport module.

4. The assembled analysis system of claim 1, wherein the status information is identified from at least one of the presence or absence of the file, a file name of the file, and content of the file.

5. The assembled analysis system of claim 1, wherein the presence or absence of the file, a file name of the file, or content of the file is updated depending on a position or a status of the transport module.

6. The assembled analysis system of claim 1, wherein content of the file includes a history of changes in a position or status of the transport module.

7. The assembled analysis system of claim 1, wherein
the file is stored in a predetermined position in the system control module, and
the driving software accesses the position using the file reading API and then reads the file stored in the predetermined position.

8. The assembled analysis system of claim 1, wherein the file has a predetermined file name, and the file is recognized by the file name.

9. The assembled analysis system of claim 1, wherein
the status information includes information on whether the transport module is ready to receive the analytical sample vessel from the preparation device, and
when the transport module is ready to receive the analytical sample vessel, the analytical sample vessel is then delivered from the preparation device to the transport module.

10. The assembled analysis system of claim 1, wherein
the status information includes information on whether the analytical sample vessel has been delivered from the preparation device to the transport module, and
the transport module transports the analytical sample vessel after the analytical sample vessel is transported from the preparation device to the transport module.

11. The assembled analysis system of claim 1, wherein the preparation device detects whether the file is stored in the system control module using a polling method or an interrupt method.

12. The assembled analysis system of claim 1, wherein the preparation device is an approved device as an independently operated device.

13. The assembled analysis system of claim 1, further comprising;
a sealing device for sealing an internal space of the analytical sample vessel against an outside of the analytical sample vessel, and the internal space accommodates the analysis target sample.

14. The assembled analysis system of claim 13, wherein the sealing device is controlled by the system control module for its operation.

15. The assembled analysis system of claim 1, further comprising:
an analysis device for performing an analysis operation on a target nucleic acid molecule for the analysis target sample.

16. The assembled analysis system of claim 15, wherein
the analysis device performs a polymerase chain reaction, and
the analysis operation includes an analysis for a result of the polymerase chain reaction.

17. A method of obtaining status information of a transport module performed in an assembled analysis system including a preparation device, the transport module, and a system control module,
wherein the preparation device prepares an analysis target sample in an analytical sample vessel,
the transport module transports the analytical sample vessel in which the analysis target sample is prepared, and
driving software of the preparation device is installed in the system control module, and a file in which the status information of the transport module is recorded is stored in the system control module, and
wherein the method of obtaining the status information of the transport module comprises
reading the file stored in the system control module using a file reading application programming interface (API) provided in the driving software; and
identifying the status information from the read file.

18. A computer program, stored in a computer-readable recording medium, programmed to perform each step included in the method according to claim 17.

19. A computer-readable recording medium storing a computer program programmed to perform each step included in the method according to claim 17.

20. An assembled analysis system comprising:
a preparation device for preparing an analysis target sample in an analytical sample vessel;
an analysis device for performing an analysis operation on the prepared analysis target sample; and
a system control module in which driving software of the preparation device and driving software of the analysis device are installed and a file is stored,
wherein a file generated in the driving software of the preparation device is recorded in the system control module by a file writing API provided in the driving software of the preparation device, and
the file recorded in the system control module is provided to the driving software of the analysis device by a file reading API provided in the driving software of the analysis device.

21. The assembled analysis system of claim 20, wherein the preparation device prepares the analysis target sample by dispensing at least one of a specimen and a reagent, and accommodates the prepared analysis target sample in the analytical sample vessel.

22. The assembled analysis system of claim 20, wherein
the analysis device performs a polymerase chain reaction, and
the analysis operation includes an analysis operation of a result of the polymerase chain reaction.

23. The assembled analysis system of claim 20, wherein the file includes an instruction used when the analysis device analyzes the analysis target sample.

24. The assembled analysis system of claim 23, wherein
the instruction includes at least one of a reaction protocol, information on a plate on which at least one analytical sample vessel is located, information on a specimen accommodated in each of the at least one analytical sample vessel, information on a reagent accommodated in each of the at least one analytical sample vessel, and information on an optical scan method performed in the analysis device.

25. The assembled analysis system of claim 20, wherein the analysis device detects whether or not the file is stored in the system control module using a polling method or an interrupt method.

26. The assembled analysis system of claim 20, wherein each of the preparation device and the analysis device is an approved device as an independently operated device.

27. A file transfer method performed in an assembled analysis system including a preparation device, an analysis device, and a system control module,
wherein the preparation device performs a preparation operation to detect a target nucleic acid molecule,
the analysis device performs an analysis operation on the target nucleic acid molecule, and
driving software of the preparation device and driving software of the analysis device are installed in the system control module, and a file is to be stored in the system control module, and
wherein the file transfer method comprises:
generating a file in the driving software of the preparation device;
recording the generated file in the system control module using a file writing application programming interface (API) provided in the driving software of the preparation device; and
providing the recorded file to the driving software of the analysis device through a file reading API provided in the driving software of the analysis device.

28. A computer program, stored in a computer-readable recording medium, programmed to perform each step included in the method according to claim 27.

29. A computer-readable recording medium storing a computer program programmed to perform each step included in the method according to claim 27.
